# DEMANDE DE BREVET EUROPEEN

(11) **EP 1 438 947 A2**
(43) Date de publication de la demande: **21.07.2004**
(21) Numéro de dépôt: 03292896.2
(22) Date de dépôt: 21.11.2003
(51) Int. Cl.: A61K 7/06, A61K 7/50

(54) **Compositions cosmétiques contenant un tensioactif amphotère et une silicone et leurs utilisations**

(30) Priorité: 19.12.2002 FR 0216192
(71) Demandeur: L'OREAL, 75008 Paris (FR)
(72) Inventeur: Gawtrey, Jonathan, 92100 Boulogne (FR); Maubru, Mireille, 78400 Chatou (FR); Pinel, Nathalie, 74160 Neydens (FR)
(74) Mandataire: Le Blainvaux Bellegarde, Françoise

(57) **Abrégé**

L'invention concerne de nouvelles compositions cosmétiques détergentes comprenant dans un milieu cosmétiquement acceptable au moins un tensioactif amphotère choisi parmi les composés alcoylamphohydroxyalkylsulfonates et leurs sels, et au moins une silicone.

Cette association apporte des propriétés cosmétiques (lissage, légèreté, douceur) sans phénomène de regraissage des fibres kératiniques.

Ces compositions sont utilisées notamment pour le lavage et/ou le conditionnement des matières kératiniques telles que les cheveux ou la peau.

## Description

La présente invention concerne de nouvelles compositions cosmétiques comprenant dans un milieu cosmétiquement acceptable au moins un tensioactif amphotère choisi parmi les composés alcoylamphohydroxyalkylsulfonates et leurs sels et au moins une silicone particulière.

Pour le nettoyage et/ou le lavage des cheveux et/ou de la peau, l'utilisation de compositions détergentes (shampooing ou gel-douche) à base essentiellement d'agents tensioactifs classiques de type notamment anionique, non ionique et/ou amphotère, mais plus particulièrement de type anionique, est courante. Ces compositions sont appliquées sur cheveux ou peau mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur les matières kératiniques des dommages plus ou moins marqués liés en particulier à l'élimination progressive des lipides ou protéines contenus dans ou à la surface de ces dernières.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés siliconés, qui confèrent en effet aux cheveux lavés, secs ou mouillés, une facilité de démêlage, une douceur et un lissage accrus par rapport à ce qui peut être obtenu avec les compositions nettoyantes correspondantes qui en sont exemptes.

Cependant, dans le cas de cheveux sensibilisés, les cheveux lavés avec un shampooing contenant des silicones sont raides c'est à dire manquent de souplesse ou de malléabilité.

Ainsi, et malgré les progrès réalisés récemment dans le domaine des shampooings à base de polymères cationiques et/ou de silicone, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoquées ci-avant, de meilleures performances.

Les tensioactifs amphotères alcoylamphohydroxyalkylsulfonates ont déjà été préconisés dans des compositions cosmétiques détergentes. Ils ont été décrits par exemple dans la demande de brevet WO99/36054.

Les compositions de lavage des cheveux utilisant ces tensioactifs seuls ne conduisent pas à de propriétés cosmétiques satisfaisantes.

L'invention a donc pour but de proposer des compositions cosmétiques détergentes présentant des propriétés cosmétiques améliorées, en particulier le démêlage, le lissage, la souplesse, la malléabilité et la douceur des cheveux.

Or, la demanderesse a maintenant trouvé que l'association de silicones particulières et d'un tensioactif amphotère alcoylamphohydroxyalkylsulfonate permettait d'atteindre ces buts.

Ces nouvelles compositions permettent également de mieux déposer ces silicones sur les matières kératiniques (notamment sur les cheveux) qu'une composition contenant des tensioactifs amphotères classiques tels que les sels de cocoamphodiacétate.

Les compositions conformes à l'invention confèrent aux matières kératiniques notamment les cheveux, un remarquable effet traitant qui se manifeste notamment par une facilité de démêlage, ainsi qu'un apport de légèreté, de lissage, de douceur, de malléabilité et de souplesse sans aucune sensation de toucher chargé.

L'invention a ainsi pour objet une composition cosmétique comprenant, dans un milieu cosmétiquement acceptable, au moins un tensioactif amphotère alcoylamphohydroxyalkylsulfonate et au moins une silicone choisie parmi les silicones non organomodifiées de viscosité allant de 500 mm²/s (500 centistokes) à 1.000.000 mm²/s (1.000.000 centistokes) et les silicones organomodifiées.

Un autre objet de l'invention concerne l'utilisation d'au moins un tensioactif amphotère tel que défini ci-dessus dans, ou pour la fabrication d'une composition cosmétique comprenant au moins une silicone telle que définie ci-dessus.

Un autre objet de l'invention concerne un procédé de traitement des matières kératiniques, telles que les cheveux, caractérisé en ce qu'il consiste à appliquer sur lesdites matières des compositions cosmétiques selon l'invention.

L'invention a encore pour objet l'utilisation d'une composition selon l'invention pour améliorer le démêlage ou le lissage des cheveux, ou pour apporter du volume, de la légèreté, de la douceur, de la souplesse ou de la malléabilité aux cheveux.

Selon la présente invention, par matières kératiniques, on comprend les cheveux, les cils, les sourcils, la peau, les ongles, les muqueuses ou le cuir chevelu et plus particulièrement les cheveux.

Les différents objets de l'invention vont maintenant être détaillés. L'ensemble des significations et définitions des composés utilisés dans la présente invention données ci-dessous sont valables pour l'ensemble des objets de l'invention.

Les tensioactifs amphotères alcoylamphohydroxyalkylsulfonates peuvent avoir la formule suivante (I) : 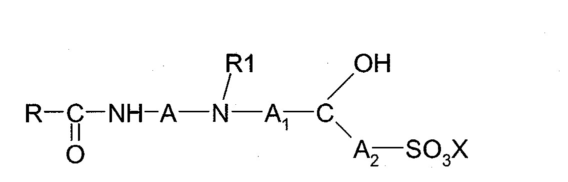 dans laquelle :
R désigne un radical hydrocarboné, saturé ou insaturé, linéaire ou ramifié comportant de 5 à 29 atomes de carbone.
R désigne de préférence un radical alkyle ou alkényle mono ou polyinsaturé comportant de 5 à 29 atomes de carbone et de préférence de 7 à 22 atomes de carbone et encore plus particulièrement de 9 à 17 atomes de carbone.
R1 désigne un radical hydroxyalkyle en C₁-C₄, de préférence hydroxyéthyle,
A, A1, A2 , identiques ou différents, désignent un radical alkylène divalent linéaire ou ramifié en C₁-C₁₀, de préférence en C₁-C₃
X désigne un atome d'hydrogène, un cation minéral ou organique tel que:
   celui d'un métal alcalin (par exemple Na⁺, K⁺) , celui d'un métal alcalinoterreux (Mg²⁺, Ca²⁺) , l'ion NH₄⁺ , les ions ammoniums issus des aminoacides basiques ou des amino-alcools.

Des composés préférés selon la présente invention sont des composés de formule (I) dans laquelle R désigne plus particulièrement un radical alkyle saturé, linéaire ou ramifié comportant de 7 à 29 atomes de carbone, de préférence de 7 à 22 atomes de carbone.

Lorsque X désigne un ion ammonium issu d'une alcanolamine, celle-ci peut être la monoéthanolamine, la diéthanolamine, la triéthanolamine, l'amino-3 propanediol-1,2. Lorsque X désigne un ion ammonium issu d'une amine, cette amine peut être un aminoacide basique tel que la lysine, l'arginine, la sarcosine, l'ornithine ou la citrulline.

De préférence, A= A2 et désigne -CH₂CH₂-.

De préférence, A1 désigne -CH2-

De préférence X désigne Na⁺

Parmi les tensioactifs de formule (I), on peut citer plus particulièrement les sels de Cocoyl amphohydroxypropyl sulfonate et notamment le sel de sodium tel que le produit proposé sous la dénomination MIRANOL CSE par la société RHODIA CHIMIE ou les sels de palmitoyl amphohydroxypropyl sulfonate et notamment le sel de sodium tel que le produit proposé sous la dénomination MIRANOL CS par la société RHODIA CHIMIE.

Selon l'invention, le ou les tensioactifs amphotères choisis parmi les composés alcoylamphohydroxyalkylsulfonates et leurs sels peuvent représenter de 0,1 % à 30 % en poids, de préférence de 1% à 20% en poids et plus particulièrement de 1,5 % à 15 % en poids, plus particulièrement 2 % à 8% en poids par rapport au poids total de la composition finale.

Les silicones utilisables conformément à l'invention peuvent être solubles ou insolubles dans l'eau ou dans la composition finale. Selon un mode préférée de l'invention, les compositions comprennent au moins une silicone insoluble dans la composition.

Les silicones insolubles sont notamment dispersés dans les compositions sous forme de particules ayant généralement une taille moyenne en nombre comprise entre 2 nanomètres et 100 microns, de préférence entre 20 nanomètres et 20 microns (mesurée avec un granulomètre).

Dans tout ce qui suit ou qui précède, on entend désigner par silicone ou polysiloxane, en conformité avec l'acceptation générale, tout polymère ou oligomère organosilicié à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydroxyls ou des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle (silicone non organomodifiée); d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyalkyls, les groupements amides, les radicaux acyloxy ou acyloxyalkyls, des groupements anioniques tels que carboxylates, thioglycolates, sulfosuccinates, thiosulfates, phosphates et sulfates, cette liste n'étant bien entendu nullement limitative (silicones dites "organomodifiées").

Par silicone non organomodifiée, on comprend au sens de la présente invention, un polysiloxane comportant sur les atomes de silicium des groupements hydroxy ou des groupements alkyles, alcényles (en particulier vinyle) ou aryles. Ces groupement étant non substitués.

Les silicones non organomodifiées de viscosité allant de 500 mm²/s (500 centistokes) à 1.000.000 mm²/s (1.000.000 centistokes) conformément à l'invention peuvent être en particulier des polysiloxanes insolubles dans la composition. Ces silicones peuvent se présenter sous forme d'huiles, de cires, de résines ou de gommes.

Les silicones non organomodifiées ont de préférence une viscosité allant de 5000 à 750.000 mm²/s à 25°C.
La viscosité de ces silicones est mesurée à 25°C selon la norme ASTM 445 Appendice C.

Les silicones insolubles dans l'eau sont insolubles dans l'eau à une concentration supérieure ou égale à 0,1% en poids dans l'eau à 25°C, c'est à dire qu'elles ne forment pas à l'oeil nu une solution isotrope transparente.

Les organopolysiloxanes sont définis plus en détail dans l'ouvrage de Walter NOLL "Chemistry and Technology of Silicones" (1968) Academie Press.

On utilise de préférence des silicones non volatiles et plus particulièrement
(i) les polydialkylsiloxanes ;
(ii) les polydiarylsiloxanes ;
(iii) les polyalkylarylsiloxanes ;
(iv) les gommes de silicone ;
(v) les résines de silicone ;
(vi) ou leurs mélanges.

Les radicaux alkyles comportent notamment de 1 à 10 atomes de carbone et désignent en particulier méthyle. Les radicaux aryles désignent plus particulièrement phényle.

Ces silicones sont plus particulièrement choisies parmi les polydialkylsiloxanes parmi lesquels on peut citer principalement les polydiméthylsiloxanes à groupements terminaux triméthylsilyle ou diméthylsilanol.

Parmi les polydialkylsiloxanes, on peut citer principalement :
- les polydiméthylsiloxanes linéaires à groupements terminaux triméthylsilyle et ayant une viscosité de 5000 à 750.000 mm²/s à 25°C, comme par exemple, et à titre non limitatif, les huiles SILBIONE® de la série 70047 commercialisées par RHODIA CHIMIE, notamment l'huile SILBIONE® 70047 V 500 000 de RHODIA CHIMIEou le polydiméthylsiloxane de viscosité 300 000 cSt, vendu sous la dénomination DC 200 fluid 300 000 par la société DOW CORNING , les huiles de la série 200 de la société DOW CORNING telles que plus particulièrement la DC200 de viscosité 60 000 Cst ;
- les polydiméthylsiloxanes linéaires à groupements terminaux hydroxydiméthylsilyle (Dimethiconol selon la dénomination CTFA) tels que les huiles de la série 48 V de RHODIA CHIMIE.
Dans cette classe de polyalkylsiloxanes, on peut également mentionner les polyalkylsiloxanes vendus par la société GOLDSCHMIDT sous les dénominations commerciales ABILWAX® 9800 et ABILWAX® 9801 qui sont des polyalkyl(C₁-C₂₀)siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 500 à 1000000 mm²/s à 25°C.

Parmi ces polyalkylarylsiloxanes on peut citer à titre d'exemple les produits commercialisés sous les dénominations suivantes :
. les huiles SILBIONE de la série 70 641 de RHONE POULENC ;
. les huiles des séries RHODORSIL 70 633 et 763 de RHONE POULENC ;
. l'huile DOW CORNING 556 COSMETIC GRAD FLUID de DOW CORNING ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Les gommes de silicone, conformes à l'invention, sont des polyorganosiloxanes de masse moléculaire moyenne en nombre comprise entre 200.000 et 1.000.000, utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On cite, par exemple, les composés suivants :
- polydiméthylsiloxane,
- poly[(diméthylsiloxane)/(méthylvinylsiloxane)],
- poly[(diméthylsiloxane)/(diphénylsiloxane)],
- poly[(diméthylsiloxane)/(phénylméthylsiloxane)],
- poly[(diméthylsiloxane)/(diphénylsiloxane)/(méthylvinylsiloxane)],

Des produits plus particulièrement utilisables conformément à l'invention sont des mélanges tels que :
. les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
. les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
. les mélanges de deux PDMS de viscosités différentes, et plus particulièrement d'une gomme PDMS et d'une huile PDMS, tels que le produit SF 1236 de la société GENERAL ELECTRIC.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ et plus particulièrement des groupements aminoalkyl(C₁-C₄) iminoalkyles(C₁-C₄);
- des groupements ammonium quaternaires comme les produits commercialisés sous les dénominations ABILQUAT 3272 et ABILQUAT 3474 par la société GOLDSCHMIDT ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334
- des groupements acyloxyalkyle tels que par exemple les polyorganosiloxanes décrits dans le brevet US-A-4957732
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Les copolymères blocs ayant un bloc linéaire polysiloxane-polyoxyalkylène de type (A-B)ₙ ayant de préférence la formule générale suivante :

([Y(R₂SiO)ₐR'₂SiYO] [(CₙH₂ₙO)_{b}])_{c} (V)

dans laquelle :
- R et R', identiques ou différents, représentent un radical hydrocarboné monovalent ne contenant pas d'insaturation aliphatique,
- n est un nombre entier allant de 2 à 4,
- a est un nombre entier supérieur ou égal à 5, de préférence compris entre 5 et 200 et encore plus particulièrement entre 5 et 100.
- b est un nombre entier supérieur ou égal à 4, de préférence compris entre 4 et 200 et encore plus particulièrement entre 5 et 100.
- c est un nombre entier supérieur ou égal à 4, de préférence compris entre 4 et 1000 et encore plus particulièrement entre 5 et 300.
- Y représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre environ 400 et environ 10.000, celui de chaque bloc polyoxyalkylène étant compris entre environ 300 et environ 10.000,
- les blocs siloxane représentent de 10 % environ à 95 % environ en poids du copolymère bloc,
- le poids moléculaire moyen en poids du copolymère bloc étant d'au moins 3.000 et de préférence compris entre 5000 et 1000000 et encore plus particulièrement entre 10000 et 200000.

R et R' sont préférentiellement choisis parmi le groupe comprenant les radicaux alkyls comme par exemple les radicaux méthyle, éthyle, propyle, butyle, pentyle, hexyle, octyle, décyle, dodécyle, les radicaux aryles comme par exemple phényle, naphtyle, les radicaux aralkyles comme par exemple benzyle, phényléthyle, les radicaux tolyle, xylyle et cyclohexyle.

Y est de préférence -R"-, -R"-CO-, -R"-NHCO-, -R"-NH-CO-NH-R"'-NHCO―, - R"-OCONH-R"'-NHCO-, où R" est un groupe alkylène divalent comme par exemple l'éthylène, le propylène ou le butylène et R"' est un groupe alkylène divalent ou un groupe arylène divalent comme -C₆H₄-, -C₆H₄-C₆H₄-, -C₆H₄-CH₂-C₆H₄-, -C₆H₄-C(CH₃)₂-C₆H₄-.

Encore plus préférentiellement, Y représente un radical alkylène divalent, plus particulièrement le radical -CH₂-CH₂-CH₂- ou le radical C₄H₈.

La préparation des copolymères blocs mis en oeuvre dans le cadre de la présente invention est décrite dans la demande européenne EP 0 492 657 A1, dont l'enseignement est inclus à titre de référence dans la présente description.

Selon l'invention, on peut encore utiliser comme silicone organomodifié des polyuréthanes siliconés tels que ceux décrits dans les demandes de brevet EP 0 751 162, EP 0 619 111 et EP1025833.

Selon l'invention, on peut également utiliser des silicones organomodifiées comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 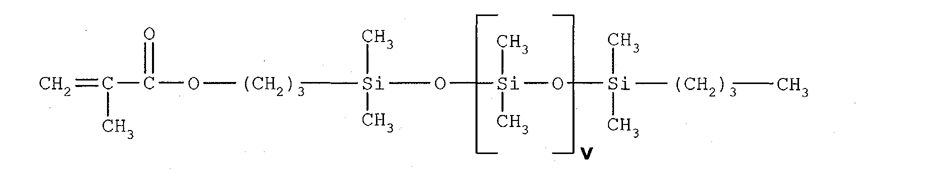 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement notamment de type thiopropylène, des motifs polymères mixtes du type acide poly(méth)acrylique et du type poly(méth)acrylate d'alkyle (par ex en C1-C4) et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions, de nanoémulsions ou de micrémulsions.

Les polyorganosiloxanes particulièrement préférés conformément à l'invention sont :
- les silicones choisies dans la famille des polydiméthylsiloxanes à groupements terminaux triméthylsilyle telles que les huiles de la séries DC200 de DOW CORNING en particulier celle de viscosité 60 000 cSt, des séries SILBIONE® 70047 et 47 et plus particulièrement l'huile SILBIONE® 70 047 V 500000 commercialisées par la société RHODIA CHIMIE, ou l'huile de silicone AK 300.000 de la société WACKER, les polydiméthylsiloxanes à groupements terminaux diméthylsilanol tels que les diméthiconols ou les polyalkylarylsiloxanes tels que l'huile SILBIONE 70641 V 200 commercialisée par la société RHONE POULENC ;
- la résine d'organopolysiloxane commercialisée sous la dénomination DOW CORNING 593 ;
- les polysiloxanes à groupements aminés tels que les amodiméthicones ou les triméthylsilylamodiméthicone.

Selon l'invention, la ou les silicones peuvent représenter de 0,001 % à 15 % en poids, de préférence de 0,01 % à 10% en poids et plus particulièrement de 0,1 à 5% en poids par rapport au poids total de la composition finale.

De préférence, le rapport en poids tensioactif(s) amphotère(s) sulfonate de l'invention / silicone de l'invention est compris entre 0,1 et 15 et plus particulièrement entre 0,5 et 10.

Les compositions de l'invention contiennent en outre avantageusement au moins un autre agent tensioactif qui est généralement présent en une quantité comprise entre 0,1% et 40% en poids environ, de préférence entre 3% et 30% et encore plus préférentiellement entre 5% et 20%, par rapport au poids total de la composition.

Cet agent tensioactif peut être choisi parmi les agents tensioactifs anioniques, amphotères, non-ioniques, ou leurs mélanges.

Les tensioactifs additionnels convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.
Parmi les tensioactifs anioniques, on préfère utiliser selon l'invention les sels d'alkylsulfates et d'alkyléthersulfates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères additionnels, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂-CONHCH₂CH₂-N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide carboxylique présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 7ème édition, 1997, sous les dénominations Disodium Cocoamphodiacetate, Disodium Lauroamphodiacetate, Disodium Capryloamphodiacetate, Disodium Caproamphodiacetate, Disodium Cocoamphodipropionate, Disodium Lauroamphodipropionate, Disodium Caproamphodipropionate, Disodium Capryloamphodipropionate, Lauroamphodipropionic acid, Cocoamphodipropionic acid.
A titre d'exemple on peut citer le disodium cocoamphodiacetate commercialisé sous la dénomination commerciale MIRANOL® C2M concentré par la société RHODIA CHIMIE.

Dans les compositions conformes à l'invention, on utilise de préférence des mélanges d'agents tensioactifs et en particulier des mélanges d'agents tensioactifs anioniques ou des mélanges d'agents tensioactifs anioniques et d'agents tensioactifs amphotères ou non ioniques.

On utilise de préférence comme agent tensioactif anionique les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélanges.

Le(s) agent(s) tensioactif(s) anionique(s) sont généralement présents à raison de 1 à 30 % en poids, de préférence de 3 à 20 % en poids, par rapport au poids total de la composition.

Le(s) agent(s) tensioactif(s) amphotère(s) (différents des composés sulfonates) ou non ioniques sont généralement présents à raison de 0,5 à environ 15% en poids, de préférence de 1 à 8% en poids, par rapport au poids total de la composition.

La quantité et la qualité des tensioactifs sont celles suffisantes pour conférer à la composition finale un pouvoir moussant et/ou détergent satisfaisant.

Dans la composition selon la présente invention, la totalité des tensioactifs détergents représente généralement de 4 à 50% en poids et de préférence de 6 à 35% en poids et plus particulièrement de 8 à 25% en poids par rapport au poids total de la composition.

La composition de l'invention peut également contenir au moins un additif choisi parmi les épaississants, les parfums, les agents nacrants, les conservateurs, les filtres solaires, les tensioactifs cationiques, les polymères anioniques ou non ioniques ou amphotères, les polymères cationiques, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, les provitamines telles que le panthénol, les huiles végétales, les huiles minérales et les huiles de synthèse, les agents antipelliculaires et tout autre additif classiquement utilisé dans le domaine cosmétique qui n'affecte pas la stabilité et les propriétés des compositions selon l'invention.

Ces additifs éventuellement sont présents dans la composition selon l'invention dans des proportions pouvant aller de 0,001 à 50% en poids par rapport au poids total de la composition. La quantité précise de chaque additif est déterminée facilement par l'homme du métier selon sa nature et sa fonction.

Selon un mode préféré de l'invention, les compositions selon l'invention comprennent en outre un ou plusieurs polymères cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société AMERCHOL, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société ONDEO, les polysaccharides cationiques non cellulosiques dont plus particulièrement les gommes de guar modifiées par du chlorure de 2,3-époxypropyl triméthylammonium commercialisées par exemple sous la dénomination « JAGUAR C13S » par la société RHODIA CHIMIE.
On peut également utiliser les polymères comprenant des motifs récurrents répondant à la formule : 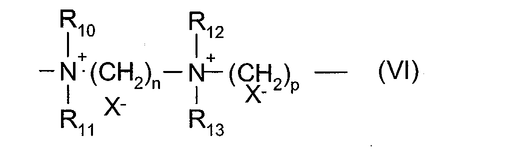 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

Le milieu cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.
De préférence , la composition comprend de 50 à 95 % en poids d'eau par rapport au poids total de la composition.

Les compositions détergentes selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants et/ou opacifiants bien connus dans l'état de la technique tels que par exemple les oxydes de titane enrobés ou non, les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses ( par exemple en C8-C30) tels que par exemple le distéaryléther ou le 1-(hexadécyloxy)-2-octadécanol, les cyclodextrines, les alcools gras tels que les alcools cétylique, stéarylique et béhénylique.

Les compositions selon l'invention peuvent contenir également des synergistes de mousses tels que des 1,2-alcanediols en C₁₀-C₁₈ ou des alcanolamides gras dérivés de mono ou de diéthanolamine.

Les compositions conformes à l'invention peuvent être utilisées pour le lavage et le traitement des matières kératiniques telles que les cheveux, la peau, les cils, les sourcils, les ongles, les lèvres, le cuir chevelu et plus particulièrement les cheveux.

En particulier, les compositions détergentes selon l'invention sont des shampooings, des gels-douche et des bains moussants.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de produits démaquillants.

Les compositions selon l'invention peuvent se présenter sous forme de gel, de lait, de crème, d'émulsion, de lotion épaissie ou de mousse et être utilisées pour la peau, le cuir chevelu, les ongles, les cils, les lèvres et plus particulièrement les cheveux.

Ces compositions détergentes sont de préférence moussantes et le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombent dans une éprouvette ayant un diamètre de 50 mm et contenant 50 ml de la composition à tester. La mesure est faite 5 minutes après l'arrêt de l'écoulement de la composition.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage en particulier avec de l'eau.

Ainsi, ce procédé selon l'invention permet le traitement, le soin, le lavage ou le démaquillage de la peau, des cheveux ou de toute autre matière kératinique.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits. Dans les exemples, MA signifie matière active.

### EXEMPLE 1 :

On a réalisé deux compositions de shampooings, l'une conforme à l'invention (composition A) et l'autre comparative (composition B) :

| Composition | Invention (A) | Comparatif (B) |
|---|---|---|
| Cocoyl amphohydroxypropyl sulfonate de sodium, en solution aqueuse à 32% de matière active, proposé sous la dénomination MIRANOL CSE par la société RHODIA | 12 g M.A. | - |
| Cocoamidopropyl bétaïne en solution aqueuse à 30% de matière active | - | 12 g M.A. |
| Polydiméthylsiloxane de viscosité 500 000 cSt, vendue sous la dénomination Mirasil DM 500.000 par la société RHODIA | 1 g | 1 g |
| Mélange 1-(hexadécyloxy) 2-octadécanol / alcool cétylique | 2.5 g | 2.5 g |
| Monoisopropanolamide d'acides de coprah | 2 g | 2 g |
| Conservateurs | q.s. | q.s. |
| Parfum | q.s. | q.s. |
| Acide citrique ou soude q.s.p. | pH 7 | pH 7 |
| Eau déminéralisée qsp | 100 g | 100 g |

On effectue un shampooing en appliquant environ 6 g de la composition A sur une demi-tête de cheveux naturels préalablement mouillés. On fait mousser le shampooing puis on rince abondamment à l'eau. On sèche les cheveux avec un sèche-cheveux.
On procède selon le même mode opératoire que ci-dessus avec la composition comparative B sur l'autre demi-tête.
Les experts comparent les demi-têtes deux à deux.

Un panel d'experts a évalué l'aspect des cheveux séchés et a noté une plus grande facilité de démêlage et un plus grand lissage au toucher,avec la composition conforme à l'invention.

Les cheveux traités avec la composition A sont significativement plus lisses et se démêlent plus facilement, que ceux traités avec la composition B.

### EXEMPLES 2 et 3

On a préparé les compositions de shampooing suivantes :

| Composition | Exemple 2 | Exemple 3 |
|---|---|---|
| Lauryléthersulfate de sodium (C12/C14 à 70/30) à 2.2 moles d'oxyde d'éthylène | 12 g M.A. | 15.5 g M.A. |
| Cocoyl amphohydroxypropyl sulfonate de sodium, en solution aqueuse à 32% de matière active, proposé sous la dénomination Miranol CSE par la société RHODIA | 1.8 g M.A. | 2.2 g M.A. |
| Hydroxyéthylcellulose réticulée par l'épichlorhydrine et quaternisée par la triméthylamine, vendue sous la dénomination JR 400 par la société AMERCHOL | 0.4 g | - |
| Gomme de guar modifiée par du chlorure de 2,3-époxypropyl triméthyl ammonium vendue sous la dénomination Jaguar C13 S par la société RHODIA | - | 0.1 g |
| Polydiméthylsiloxane de viscosité 500 000 cSt, vendu sous la dénomination Mirasil DM 500 000 par la société RHODIA | 1 g | - |
| Polydiméthylsiloxane de viscosité 300 000 cSt, vendu sous la dénomination DC 200 fluid 300 000 par la société DOW CORNING | - | 2.7 g |
| Mélange 1-(hexadécyloxy) 2-octadécanol / alcool cétylique | 2.5 g | - |
| Distéaryl éther | - | 1.5 g |
| Alcool béhénylique | - | 1.5 g |
| Mono-laurate de sorbitan oxyéthyléné à 4 moles d'oxyde d'éthylène, commercialisé sous la dénomination Tween 21 par la société UNIQEMA | 8 g | - |
| Monoisopropanolamide d'acides de coprah | 0.1 g | 1 g |
| Acide polyacrylique réticulé | 0.2 g | 0.2 g |
| Conservateurs | q.s. | q.s. |
| Parfum | q.s. | q.s. |
| Acide citrique q.s. | pH 5.3 | pH 7 |
| Eau déminéralisée q.s.p. | 100 g | 100 g |

Les cheveux lavés avec ces compositions se démêlent facilement, sont lisses (au toucher et visuellement), et malléables (souples).

### EXEMPLE 4

On a préparé la composition de shampooing suivante :

| | |
|---|---|
| Lauryléthersulfate de sodium (C12/C14 à 70/30) à 2.2 moles d'oxyde d'éthylène | 15.4 g M.A. |
| Cocoyl amphohydroxypropyl sulfonate de sodium, en solution aqueuse à 38% de matière active, commercialisé sous la dénomination Miranol CS par la société RHODIA | 2.4 g M.A. |
| Homopolymère de chlorure de diméthyl diallyl ammonium, en solution aqueuse à 40% M.A., commercialisé sous la dénomination Merquat 100 par la société NALCO | 0.48 g MA |
| Polydiméthylsiloxane à groupements aminoéthyl iminobutyl et α,ω silanols en émulsion aqueuse cationique à 60%, commercialisé sous la dénomination DC2-8299 par la société DOW CORNING | 1.2 g MA |
| Alcool béhénylique | 1.5 g |
| Distéaryl éther | 1.5 g |
| Acide polyacrylique réticulé | 0.4 g |
| Conservateurs, parfum | q.s. |
| Acide citrique q.s.p. | pH 5.1 |
| Eau déminéralisée q.s.p. | 100 g |

## Revendications

1. Composition cosmétique, **caractérisée par le fait qu'**elle comprend, dans un milieu cosmétiquement acceptable au moins un tensioactif amphotère choisi parmi les composés alcoylamphohydroxyalkylsulfonates et leurs sels et au moins une silicone choisie parmi les silicones non organomodifiées de viscosité allant de 500 mm²/s (500 centistokes) à 1.000.000 mm²/s (1.000.000 centistokes) et les silicones organomodifiées.

2. Composition selon la revendication 1, **caractérisée par le fait que** ledit tensioactif amphotère est choisi parmi les composés alcoylamphohydroxyalkylsulfonates et leurs sels ayant la formule suivante (I) : 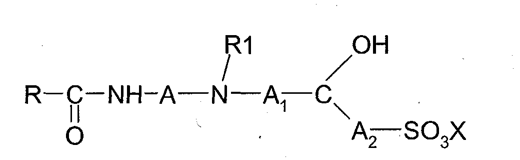 dans laquelle :
R désigne un radical hydrocarboné, saturé ou insaturé, linéaire ou ramifié comportant de 5 à 29 atomes de carbone.
R1 désigne un radical hydroxyalkyle en C₁-C₄, de préférence hydroxyéthyle,
A, A1, A2 , identiques ou différents, désignent un radical alkylène divalent linéaire ou ramifié en C₁-C₁₀, de préférence en C₁-C₃,
X désigne un atome d'hydrogène, un cation minéral ou organique.

3. Composition selon la revendication 2, **caractérisée par le fait que** R désigne de préférence un radical alkyle ou alkényle mono ou polyinsaturé comportant de 5 à 29 atomes de carbone et de préférence de 7 à 22 atomes de carbone et encore plus particulièrement de 9 à 17 atomes de carbone.

4. Composition selon l'une quelconque des revendications 2 ou 3, **caractérisée par le fait que** A et A2 désignent -CH₂CH₂-.

5. Composition selon l'une quelconque des revendications 2 à 4, **caractérisée par le fait que** A1 désigne -CH₂-.

6. Composition selon l'une quelconque des revendications 2 à 5, **caractérisée par le fait que** le cation minéral ou organique est choisi parmi celui d'un métal alcalin ( par exemple Na⁺, K⁺) , celui d'un métal alcalinoterreux (Mg²⁺, Ca²⁺), l'ion NH₄⁺ , les ions ammoniums issus des aminoacides basiques ou des amino-alcools.

7. Composition selon l'une quelconque des revendications 1 à 6, **caractérisée par le fait que** ledit tensioactif amphotère est un sel de Cocoyl amphohydroxypropyl sulfonate et les sels de palmitoyl amphohydroxypropyl sulfonate.

8. Composition selon l'une quelconque des revendications 1 à 7, **caractérisée en ce que** le ou les tensioactifs amphotères alcoylamphoallkylsulfonates et leurs sels sont présents à une concentration comprise entre 0,1 et 30 % en poids, de préférence entre 1 et 20% en poids par rapport au poids total de la composition.

9. Composition selon l'une quelconque des revendications 1 à 8, **caractérisée par le fait que** les silicones se présentent sous forme d'huiles, de cires, de résines ou de gommes.

10. Composition selon l'une quelconque des revendications 1 à 9, **caractérisée par le fait que** les silicones non organomodifiées sont des polysiloxanes choisis parmi les polydialkylsiloxanes, les polydiarylsiloxanes, les polyalkylarylsiloxanes, les gommes et résines de silicones, ainsi que leurs mélanges.

11. Composition selon la revendication 10, **caractérisée par le fait que** :
(a) les polydialkylsiloxanes sont choisis parmi :
- les polydiméthylsiloxanes à groupements terminaux triméthylsilyle ;
- les polydiméthylsiloxanes à groupements terminaux diméthylsilanol ;
- les polyalkyl(C₁-C₂₀)siloxanes ;
(b) les polyalkylarylsiloxanes sont choisis parmi :
- les polydiméthylméthylphénylsiloxanes, les polydiméthyldiphényl siloxanes linéaires et/ou ramifiés;
(c) les gommes de silicone sont choisies parmi les polydiorganosiloxanes ayant des masses moléculaires moyennes en nombre comprises entre 200 000 et 1 000 000 utilisés seuls ou sous forme de mélange dans un solvant ;
(d) les résines sont choisies parmi les résines constituées d'unités : R₃ Si O_{1/2}, R₂ Si O_{2/2}, R Si O_{3/2}, Si O_{4/2}
dans lesquelles R représente un groupement hydrocarboné ayant de 1 à 16 atomes de carbone ou un groupement phényle ;

12. Composition selon l'une quelconque des revendications 10 ou 11, **caractérisée par le fait que** les gommes de silicone utilisées seules ou sous forme de mélange sont choisies parmi les structures suivantes :
- polydiméthylsiloxane
- polydiméthylsiloxane/méthylvinylsiloxanes,
- polydiméthylsiloxane/diphénylsiloxane,
- polydiméthylsiloxane/phénylméthylsiloxane,
- polydiméthylsiloxane/diphénylsiloxane/méthylvinylsiloxanes et les mélanges suivants :
- des mélanges formés à partir d'un polydiméthylsiloxane hydroxylé en bout de chaîne et d'un polydiméthylsiloxane cyclique ;
- les mélanges formés à partir d'une gomme polydiméthylsiloxane et d'une silicone cyclique ; et
- des mélanges de polydiméthylsiloxanes de viscosités différentes.

13. Composition selon l'une quelconque des revendications 1 à 9, **caractérisée par le fait que** les silicones organomodifiées sont choisies parmi les polyorganosiloxanes comportant :
a) des groupements polyéthylèneoxy et/ou polypropylèneoxy ;
b) des groupements aminés substitués ou non ;
c) des groupements thiols ;
d) des groupements alcoxylés,
e) des groupements hydroxyalkyle,
f) des groupements acyloxyalkyle,
g) des groupements alkyl carboxyliques,
h) des groupements 2-hydroxyalkylsulfonates,
i) des groupements 2-hydroxyalkylthiosulfates,
j) des groupements hydroxyacylamino,
k) des groupements ammonium quaternaires.

14. Composition selon l'une quelconque des revendications 1 à 13, **caractérisée par le fait que** les siliconesnes sont choisis parmi les polyalkylsiloxanes à groupements terminaux triméthylsilyle, les polyalkylsiloxanes à groupements terminaux diméthylsilanol, les polyalkylarylsiloxanes, les résines d'organopolysiloxanes, les polysiloxanes à groupements aminés.

15. Composition selon l'une quelconque des revendications 1 à 14, **caractérisée par le fait que** les silicones sont choisies parmi les polyorganosiloxanes insolubles dans la composition.

16. Composition selon l'une quelconque des revendications 1 à 15, **caractérisée en ce que** le ou les tensioactifs amphotères alcoylamphoallkylsulfonates et leurs sels sont présents à une concentration comprise entre 0,1 et 30 % en poids, de préférence entre 1 et 20% en poids par rapport au poids total de la composition.

17. Composition selon l'une quelconque des revendications 1 à 16, **caractérisée en ce que** ladite silicone est présente à une concentration comprise entre 0,001 % et 15 % en poids par rapport au poids total de la composition, de préférence entre 0,01 % et 10 % en poids et en particulier entre 0,1 % et 5 % en poids.

18. Composition selon l'une quelconque des revendications précédentes, **caractérisée en ce que** le rapport en poids tensioactif(s) amphotère(s) sulfonate de l'invention / silicone de l'invention est compris entre 0,1 et 15 et plus particulièrement entre 0,5 et 10.

19. Composition selon l'une quelconque des revendications 1 à 18, **caractérisée par le fait qu'**elle comprend en outre au moins un agent tensioactif additionnel choisi parmi les tensioactifs anioniques, cationiques, non ioniques, amphotères et leurs mélanges.

20. Compositions selon la revendication 19, **caractérisées par le fait que** le ou les agents tensioactifs additionnels sont présents à une concentration comprise entre 0,1% et 40% en poids, de préférence entre 3% et 30% en poids, et encore plus préférentiellement entre 5% et 20% en poids, par rapport au poids total de la composition.

21. Composition selon l'une quelconque des revendications 1 à 20, **caractérisée par le fait qu'**elle comprend en outre au moins un additif choisi parmi les épaississants, les parfums, les agents nacrants et/ou oacifiants, les conservateurs, les filtres solaires, les polymères anioniques ou non ioniques ou amphotères, les polymères cationiques, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, les provitamines telles que le panthénol, les huiles végétales, les huiles minérales, les huiles de synthèse, les agents antipelliculaires.

22. Composition selon la revendication 21, **caractérisée par le fait que** lesdits polymères cationiques sont choisis parmi :
- les homopolymères de sel de diallyldiméthylammonium et les copolymères de sel de diallyldiméthylammonium et d'acrylamide,
- les dérivés d'éther de cellulose quaternaires,
- les polysaccharides cationiques non cellulosiques, en particulier les gommes de guar cationiques,
- les polymères comprenant des motifs récurrents répondant à la formule : 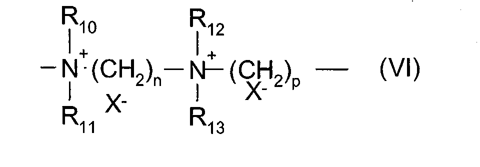
dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

23. Composition selon l'une quelconque des revendications 21 ou 22, **caractérisée en ce que** le polymère cationique est présent à une concentration comprise entre 0,001 % et 10 % en poids par rapport au poids total de la composition, de préférence entre 0,005 % et 5 % en poids.

24. Composition selon la revendication 21, **caractérisée par le fait que** d'agents nacrants et/ou opacifiants sont choisis parmi les oxydes de titane enrobés ou non, les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses ( par exemple en C8-C30) tels que par exemple le distéaryléther ou le 1-(hexadécyloxy)-2-octadécanol, les cyclodextrines, les alcools gras tels que les alcools cétylique, stéarylique et béhénylique.

25. Composition selon l'une quelconque des revendications 1 à 24, **caractérisées par le fait qu'**elle se présente sous forme de shampooing, de composition lavantes pour la peau, d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

26. Utilisation d'une composition telle que définie dans l'une quelconque des revendications précédentes pour le lavage des matières kératiniques en particulier les cheveux.

27. Utilisation d'une composition telle que définie dans l'une quelconque des revendications 1 à 24 pour améliorer le démêlage ou le lissage des cheveux , ou pour apporter du volume, de la légèreté, de la douceur, de la souplesse ou de la malléabilité aux cheveux.

28. Procédé de traitement des matières kératiniques, telles que les cheveux, **caractérisé en ce qu'**il consiste à appliquer sur lesdites matières une composition cosmétique selon l'une des revendications 1 à 24, puis à effectuer éventuellement un rinçage.
